# EUROPEAN PATENT SPECIFICATION

(11) **EP 4 433 815 B1**
(45) Date of publication and mention of the grant of the patent: **24.12.2025**
(21) Application number: 22818115.2
(22) Date of filing: 14.11.2022
(51) Int. Cl.: G01N 29/265, G01N 29/04, G01N 29/26, G01N 29/22, B61K 9/10

(54) **DEVICE AND METHOD FOR PERFORMING NON-DESTRUCTIVE CONTROL OF A RAIL**
VORRICHTUNG UND VERFAHREN FÜR DIE ZERSTÖRUNGSFREIE PRÜFUNG EINER SCHIENE
APPAREIL ET METHODE POUR EFFECTUER LE CONTROLE NON-DESTRUCTIF D'UN RAIL

(30) Priority: 16.11.2021 IT 202100029039
(43) Date of publication of application: 25.09.2024
(73) Proprietor: PCM Srl Socio Unico, 48123 Ravenna (IT)
(72) Inventor: SALCICCIA, Marco, 48121 Ravenna (IT)
(74) Representative: Roncuzzi, Davide
(86) International application number: PCT/IB2022/060916
(87) International publication number: WO 2023/089465

(56) References cited:
- CN-A- 111 337 574
- CN-U- 210 427 452
- CN-U- 212 989 244
- JP-A- H09 159 655

## Description

The present invention concerns a device for the non-destructive control of a rail. In particular, the present invention refers to a device comprising a frame and an ultrasound detection unit supported by the frame. More specifically, the present invention refers to a device also comprising movement means designed to move the detection unit with respect to the frame.

### DESCRIPTION OF THE STATE OF THE ART

In the railway superstructure sector, the need to perform periodic non-destructive controls on the state of the rails that compose the tracks has been felt for some time, in order to promptly identify any defects which in the long term could deteriorate until compromising operation of the track.

Generally, each rail is made by butt-welding a plurality of steel profiles with length between 12 metres and 108 metres, having a substantially double T-shaped section; more specifically, said section has an upper head designed to define the rolling surface for transit of the rolling stock, a lower foot resting on the track sleepers and having transverse width greater than that of the head, and a web (or core) arranged vertically to join the head and the foot. Usually, the profiles are head-joined by aluminothermic welding: this process is particularly delicate, as it is carried out *in situ* on the profiles already fixed to the sleepers, therefore outdoors and in temperature and humidity conditions which are not easy to control.

Consequently, in the context of controls on the state of the rails, the checks on the welds between the profiles are of crucial importance, since defects are often found in the area of these joints, due to the frequent sub-optimal ambient conditions in which the aluminothermic welding operation is carried out.

In the technical field of reference, different solutions are known for performing non-destructive controls on the welds between the profiles that compose the rails. The most common solution entails the use of an ultrasound probe which is positioned in contact with the surface of the rail, and moved manually by an operator along said surface: the probe is designed to emit high frequency sound waves which propagate inside the rail, and to capture said waves once they have undergone reflection. By moving the probe along the surface of the rail, and comparing the different wave reflection patterns obtained, it is possible to identify the presence of any defects in the weld.

This solution allows rapid control of the welds, at the same time guaranteeing operator safety, but it has the drawback of being unreliable and difficult to reproduce: it has been observed, in fact, that the result of the control depends to a large extent on the ability and experience of the operator who moves the probe, since the variation in the reflection pattern of the ultrasounds inside the rail is significantly influenced by the way in which the probe is moved along the relative surface. Furthermore, this solution can only be used for control of the head and web of the rail, since propagation of the sound waves in the foot produces a much more complicated, and even more dependent on the way in which the probe is moved, reflection pattern.

As an alternative to this procedure, the use of X-ray scanning devices is often proposed to detect the presence of any defects in the welds between the profiles. This solution is much more reliable and reproducible than the use of ultrasound waves, due to the high penetration capacity of the electromagnetic waves used, but poses important safety problems: the operators responsible for the controls have to be provided with adequate radiation screening equipment, while the rest of the personnel have to be moved a sufficient distance away from the area. This requirement makes the weld control procedure particularly slow and costly.

Other fairly common solutions for control of the welds between the profiles consist in the use of penetrating liquids or magnetic detectors: however, both methods only allow the identification of surface defects, or defects located just below the surface of the rail, and are ineffective in identifying in-depth defects.

An example of a device according to the pre-characterizing part of the first claim is known from the document CN 210 427 452 U.

The problem of creating a device and a procedure that allow rapid, reliable and reproducible control of the welds between the profiles composing the rails, at the same time guaranteeing the safety of the operators involved, is currently unsolved, and represents an interesting challenge for the applicant.

In the light of the situation described above, it would be desirable to have an inexpensive and practical-to-use device that can limit, and if possible overcome, the drawbacks typical of the state of the art.

### SUMMARY OF THE PRESENT INVENTION

The present invention concerns a device for the non-destructive control of a rail. In particular, the present invention refers to a device comprising a frame and an ultrasound detection unit supported by the frame. More specifically, the present invention refers to a device also comprising movement means designed to move the detection unit with respect to the frame.

The drawbacks described above are solved by the present invention according to at least one of the following claims.

According to the present invention, a device is provided for performing the non-destructive control of a first rail that develops along a first axis, said device comprising a frame designed to be moved along said first rail resting at least partially thereon, and an ultrasound detection unit supported by said frame, characterized in that it comprises movement means designed to move said detection unit with respect to said frame in a first direction substantially parallel in use to said first axis, in a second direction substantially perpendicular to said first direction and in a third direction substantially perpendicular to said first direction and second direction.

According to an embodiment as described above, said movement means comprise a cross member supported by said frame and movable with respect to said frame in said second direction, a slide supported by said cross member and movable with respect to said cross member in said first direction, a carriage supported by said slide and movable with respect to said slide in said third direction, and actuation means designed to move said cross member, said slide and said carriage, said detection unit being supported by said carriage.

According to the present invention, said detection unit comprises a support, a first ultrasound probe connected to said support and a second ultrasound probe connected to said support.

According to the present invention, said first probe and second probe are of the phased array type and have respectively a first emission direction and a second emission direction intersecting with each other. According to the present invention, said detection unit comprises a third ultrasound probe of the conventional type with a single element and/or a fourth ultrasound probe of the conventional type with a double element, said third probe and fourth probe having respectively a third emission direction and a fourth emission direction substantially perpendicular to said first direction.

According to an embodiment as described above, said first probe and/or said second probe are movable along said support.

According to an embodiment as described above, said first probe and/or said second probe are rotatable around a second axis substantially parallel to said first direction.

According to an embodiment as described above, said device comprises identification means supported by said frame and designed to identify a given position along said first rail in which to perform said control.

According to an embodiment as described above, said device comprises electronic means supported by said frame and designed to command operation of said detection unit and activation of said movement means.

According to an embodiment as described above, said electronic means are designed to receive data from said detection unit and send said data to a remote operations centre.

According to some embodiments of the present invention, a method is provided for performing a non-destructive control on a first rail which develops along a first axis, by means of a device as described above, comprising the following steps:
a. moving said device along said first rail until it reaches a position that can be defined as required;
b1. moving said detection unit along said first direction, second direction and third direction by means of said movement means to bring said first probe and second probe into contact with an upper surface of said first rail;
b2. moving said detection unit in said first direction and/or in said second direction by means of said movement means, to move said first probe and second probe along said upper surface and to perform at least a first part of said control by means of said first probe and second probe;
b3. moving said detection unit in said first direction, second direction and third direction by means of said movement means to bring said third probe and/or said fourth probe into contact with an upper surface of said first rail;
b4. moving said detection unit in said first direction and/or in said second direction by means of said movement means, to move said third probe and/or said fourth probe along said upper surface and to perform a second part and/or a third part of said control by means of said third probe and/or said fourth probe.

According to some embodiments of the present invention, a method is provided for performing the non-destructive control of a first rail according to claims 9-10.

According to some embodiments of the present invention, a method is provided for performing the non-destructive control of a rail according to claim 11.

### BRIEF DESCRIPTION OF THE FIGURES

Further characteristics and advantages of the device and the method according to the present invention will appear clearer from the following description, provided with reference to the attached figures that illustrate at least one non-limiting embodiment example thereof. In particular:
- figure 1 is a three-dimensional perspective view of a preferred non-limiting embodiment of a device according to the present invention;
- figure 2 is an enlarged three-dimensional perspective view of figure 1, from a different observation point, with parts removed for clarity;
- figure 3 is an enlarged three-dimensional perspective view of a detection unit extracted from figure 2;
- figure 4 is a lateral view of figure 3;
- figure 5 is an enlarged front view of figure 2, with parts removed for clarity.

### DETAILED DISCLOSURE OF THE PRESENT INVENTION

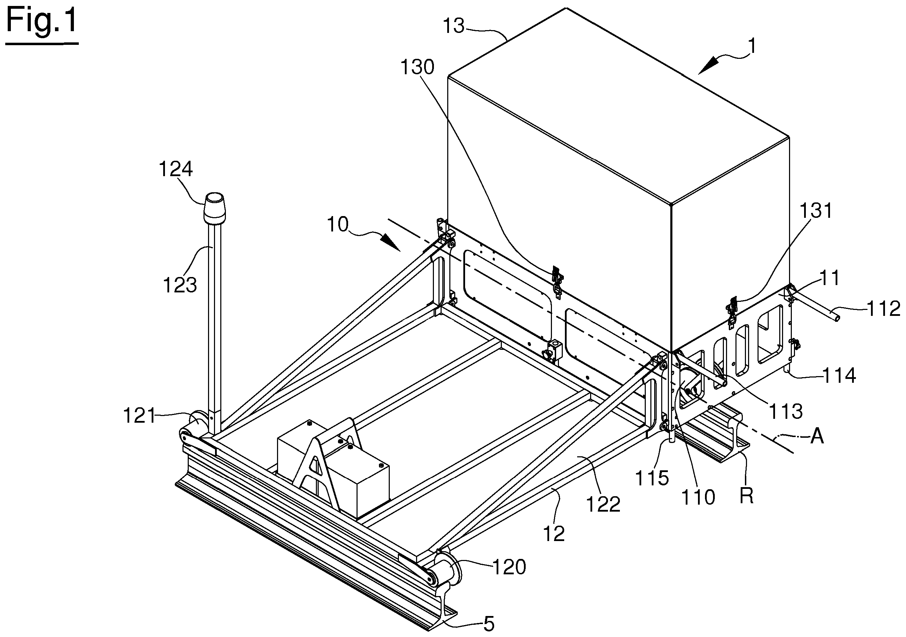

In figure 1, the number 1 indicates overall a device for performing the non-destructive control of a first rail R that develops along a first axis A. In particular, if the first rail R is rectilinear, the first axis A shall be identified by the direction in which the first rail R is arranged, whereas if the first rail R is curvilinear, the first axis A shall be identified by the direction tangent to the first rail R at the point in which the control is performed.

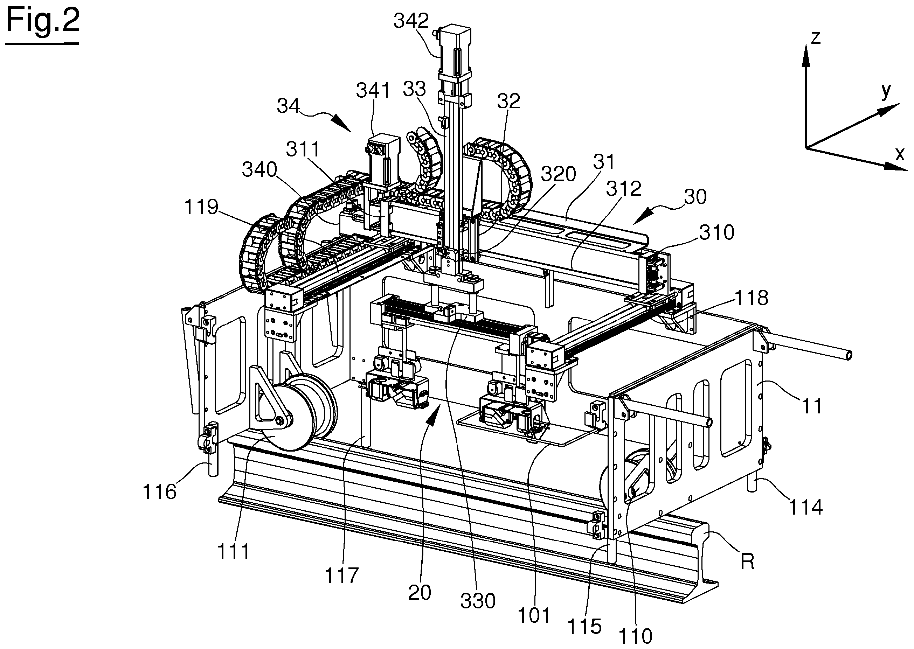

The device 1 comprises a frame 10, comprising in turn a box-like body 11 having a first wheel 110 and a second wheel 111 (shown in figure 2) arranged to rest on the first rail R so that it can roll thereon. The box-like body 11 is surmounted by a polycarbonate cover 13, selectively connectable to the box-like body 11 by means of a plurality of connection elements 130, 131: the cover 13 is designed to house and protect the most delicate components of the device 1. The box-like body 11 furthermore has a plurality of handles 112, 113 arranged to allow movement of the device 1 along the first rail R, and a plurality of support feet 114, 115, 116 and 117 (shown in figure 2) designed to support the box-like body 11 when it is not resting on the first rail R.

The frame 10 further comprises a railway plate 12 selectively connectable to the box-like body 11, having a third wheel 120 and a fourth wheel 121 designed to rest on a second rail S arranged parallel to the first rail R so as to form a railway track. The plate 12 furthermore has a transport surface 122 arranged transversely between the first rail R and the second rail S, and an upright 123 on the upper end of which a flashing warning light 124 is positioned.

With reference to figure 2, the device 1 comprises an ultrasound detection unit 20 supported by the box-like body 11 therein, and movement means 30 arranged inside the space defined by the first box-like body 11 and the cover 13 and designed to move the detection unit 20 with respect to the box-like body 11 in a first direction X substantially parallel to the first axis A, in a second direction Y substantially perpendicular to the first direction X, and in a third direction Z substantially perpendicular to the first direction X and to the second direction Y.

In particular, the box-like body 11 supports therein a first guide 118 and a second guide 119 arranged parallel to the second direction Y: the movement means 30 therefore comprise a cross member 31 which extends parallel to the first direction X, the cross member 31 having a first end 310 designed to slidingly engage the first guide 118, and a second end 311 designed to slidingly engage the second guide 119, so that the cross member 31 is movable parallel to the second direction Y.

The movement means 30 further comprise a slide 32 designed to slidingly engage a third guide 312 provided along the cross member 31, so that the slide 32 is supported by the cross member 31 and is movable with respect thereto parallel to the first direction X, and a carriage 33 designed to slidingly engage a fourth guide 320 provided along the slide 32, so that the carriage 33 is supported by the slide 32 and is movable with respect thereto parallel to the third direction Z. The carriage 33 furthermore has a plate 330 designed to be coupled to the detection unit 20, so that the detection unit 20 is moved integral with the carriage 33.

The movement means 30 lastly comprise actuation means 34 comprising a first electric motor 340 supported by the second guide 119 and designed to move the cross member 31 parallel to the second direction Y, a second electric motor 341 supported by the cross member 31 and designed to move the slide 32 parallel to the first direction X, and a third electric motor 342 supported by the carriage 33 and designed to move the carriage 33 parallel to the third direction Z.

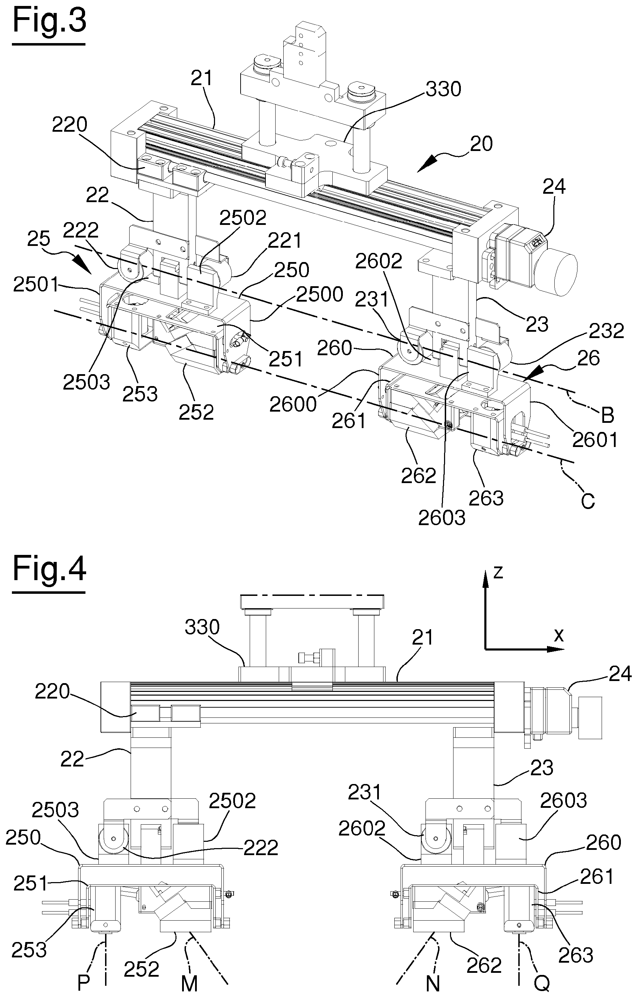

With reference to figure 3, the detection unit 20 comprises a support 21 designed to be coupled to the plate 330 of the carriage 33, and a first post 22 and a second post 23 connected to the support 21. In particular, the first post 22 is connected to the support 21 by the interposition of a cursor 220, so as to be movable along the support 21 by means of an adjustment member 24: consequently, by operating the adjustment member 24, it is possible to move, as required, the first post 22 near to or away from the second post 23, which is preferably fixed to one end of the support 21.

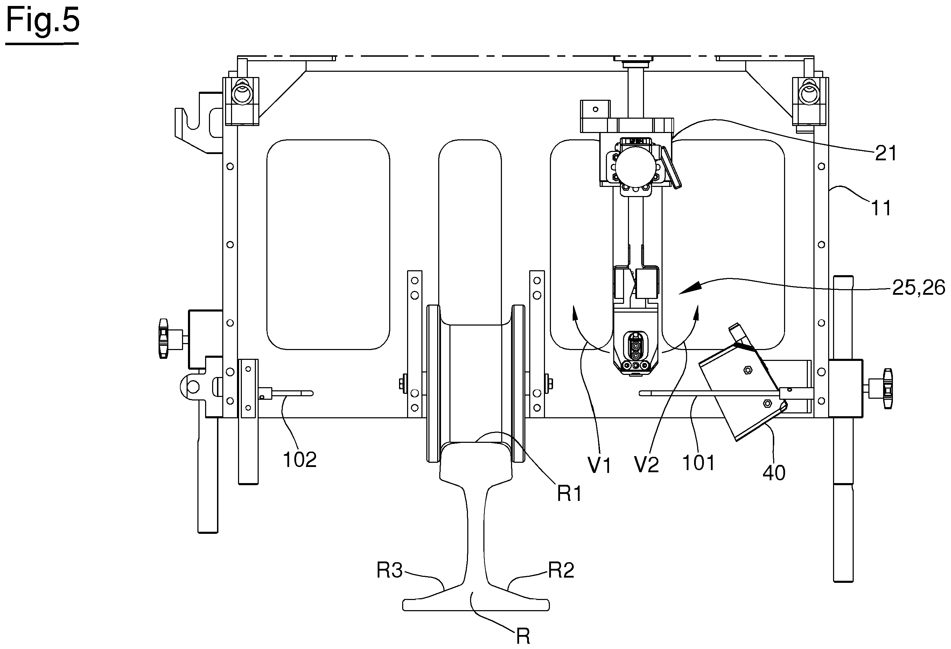

The first post 22 carries in turn a first probe assembly 25, comprising a first fork 250 connected to the first post 22 so as to be rotatable around a second axis B parallel to the first direction X in a first way V1 and a second way V2 (shown in figure 5), the first fork 250 having a first arm 2500 and a second arm 2501. The first fork 250 furthermore has a first metal contact element 2502 and a second metal contact element 2503 designed to contact respectively a first electromagnet 221 and a second electromagnet 222 integral with the first post 22, preventing rotation of the first fork 250 beyond a given angle around the second axis B. A first base 251 is hinged to the respective terminal portions of the first arm 2500 and the second arm 2501, so as to be rotatable around a third axis C parallel to the second axis B, and carries a first ultrasound probe 252 and a third ultrasound probe 253.

Analogously the second post 23 carries a second probe assembly 26, comprising a second fork 260 connected to the second post 23 so as to be rotatable around the second axis B in the first way V1 and in the second way V2, the second fork 260 having a third arm 2600 and a fourth arm 2601. The second fork 260 furthermore has a third metal contact element 2602 and a fourth metal contact element 2603 designed to contact respectively a third electromagnet 231 and a fourth electromagnet 232 integral with the second post 23, preventing rotation of the second fork 260 beyond a given angle around the second axis B. A second base 261 is hinged to the respective terminal portions of the third arm 2600 and fourth arm 2601, so as to be rotatable around the third axis C, and carries a second ultrasound probe 262 and a fourth ultrasound probe 263.

With reference to figure 4, the first probe 252 and the second probe 262 are of the phased array type. This expression indicates that the first probe 252 and the second probe 262 have respectively a first array and a second array of ultrasound wave emission sources; said source arrays are controlled so as to generate overall parallel wave fronts that are propagated respectively in a first emission direction M and a second emission direction N which can be defined as required by simply varying the sequence in which the emission sources are activated. The first emission direction M and the second emission direction N are oriented so as to be intersecting with each other.

The third probe 253 is of the conventional type with a single element. This expression indicates that the third probe 253 has one single ultrasound wave emission source, which also acts as a detection element, and therefore also has a third well-defined emission direction P. The third probe 253 is oriented so that the third emission direction P is arranged perpendicular to the first direction X, namely, in use, perpendicular to the first axis A of the first rail R.

The fourth probe 263 is of the conventional type with double element. This expression indicates that the fourth probe 263 has one single ultrasound wave emission source, and a detection element alongside said source. Consequently, also in this case a fourth emission direction Q is well defined, and in addition a depth is also defined at which the scan reaches maximum precision. The fourth probe 263 is oriented so that the fourth emission direction Q is arranged perpendicular to the first direction X, namely, in use, perpendicular to the first axis A of the first rail R.

With reference to figure 5, the box-like body 11 comprises a first abutment 101 (shown also in figure 2) and a second abutment 102: said abutments 101, 102 are designed to abut the first probe assembly 25 or the second probe assembly 26 when one of the latter is brought into contact with one of the abutments 101, 102 by the movement means 30, so as to cause a rotation of said probe assembly 25, 26 around the second axis B.

The device 1 further comprises identification means 40 supported by the box-like body 11 and designed to identify a given position in which to perform the control along the first rail R. In particular the identification means 40 comprise a laser profilometer 40, adapted to identify the central region of a weld between two profiles that compose the first rail R, in order to allow the most suitable positioning of the detection unit 20 with respect to the first rail R by means of the movement means 30.

The device 1 lastly comprises electronic control means, known and not illustrated, supported by the box-like body 11 and designed to control operation of the actuation means 34 of the movement means 30 so as to control, as required, the positioning of the detection unit 20. Furthermore, the electronic control means are designed to command and control operation of the probes 252, 253, 262, 263, to perform the control on the first rail R. The electronic control means are lastly designed to receive data from the probes 252, 253, 262, 263, and to send said data to a remote operations centre: at this remote operations centre, the data can be collected, analysed and presented to the personnel in charge, so as to allow the geographical localization of any defects present in the first rail R and provide information to the personnel concerning the operations necessary for maintenance of the railway line.

In use, the device 1 is used to actuate a method for the non-destructive control of the first rail R, comprising the following steps:
a. moving the device 1 along the first rail R until it reaches a position that can be defined as required in which to perform the control;
b. moving the detection unit 20 in the first direction X, the second direction Y and/or the third direction Z by means of the movement means 30 to perform the control of the first rail R by means of the detection unit 20.

The step "a" of the method can further comprise one or more of the following sub-steps:
a1. moving the device 1 along the first rail R until it reaches a weld between two profiles that compose the first rail R;
a2. identifying a central region of the welding, by means of the identification means 40.

The step "b" of the method can comprise one or more of the following sub-steps:
b1. moving the detection unit 20, by means of the movement means 30, to bring the first probe 252 and the second probe 262 into contact with an upper surface R1 (shown in figure 5) of the first rail R, preferably so that the central region of the weld is in an intermediate position between the first probe 252 and the second probe 262;
b2. moving the detection unit 20, by means of the movement means 30, to move the first probe 252 and the second probe 262 along the upper surface R1 and to perform a first part of the control of the first rail R by means of the first probe 252 and the second probe 262;
b3. moving the detection unit 20, by means of the movement means 30, to bring the third probe 253 into contact with the upper surface R1;
b4. moving the detection unit 20, by means of the movement means 30, to move the third probe 253 along the upper surface R1 and to perform a second part of the control of the first rail R by means of the third probe 252;
b3'. moving the detection unit 20, by means of the movement means 30, to bring the fourth probe 263 into contact with the upper surface R1;
b4'. moving the detection unit 20, by means of the movement means 30, to move the fourth probe 263 along the upper surface R1 and to perform a third part of the control of the first rail R by means of the fourth probe 262;
b5. moving the detection unit 20, by means of the movement means 30, and rotating the first probe 252 and the second probe 262 around the second axis B in the first way V1 to bring the first probe 252 and the second probe 262 into contact with a first lower surface R2 (shown in figure 5) of the first rail R;
b6. moving the detection unit 20, by means of the movement means 30, to move the first probe 252 and the second probe 262 along the first lower surface R2 and to perform a fourth part of the control of the first rail R by means of the first probe 252 and the second probe 262;
b7. moving the detection unit 20, by means of the movement means 30, and rotating the first probe 252 and the second probe 262 around the second axis B in the second way V2 to bring the first probe 252 and the second probe 262 into contact with a second lower surface R3 (shown in figure 5) of the first rail R;
b8. moving the detection unit 20, by means of the movement means 30, to move the first probe 252 and the second probe 262 along the second lower surface R3 and to perform a fifth part of the control of the first rail R by means of the first probe 252 and the second probe 262.

The step "b5" of the method can further comprise one or more of the following sub-sub-steps:
b5-a. moving the detection unit 20, by means of the movement means 30, to bring the first arm 2500 and/or the second arm 2501 of the first fork 250 into contact with the first abutment 101 so that the first fork 250 and the first abutment 101 are alongside each other parallel to the second direction Y;
b5-b. moving the detection unit 20 in the second direction Y, by means of the movement means 30, so that the contact between the first abutment 101 and the first fork 250 causes a rotation of the first fork 250 around the second axis B in the first way V1, until causing contact of the first contact element 2502 and the second contact element 2503 with the first electromagnet 221 and the second electromagnet 222 respectively;
b5-c. activating the first electromagnet 221 and the second electromagnet 222, so that the first contact element 2502 and the second contact element 2503 remain respectively locked in position adhering to the electromagnets 221, 222 due to the magnetic attraction, locking the first fork 250 in a first inclined position;
b5-d. moving the detection unit 20, by means of the movement means 30, to bring the third arm 2600 and/or the fourth arm 2601 of the second fork 260 into contact with the first abutment 101 so that the second fork 260 and the first abutment 101 are alongside each other parallel to the second direction Y;
b5-e. moving the detection unit 20 in the second direction Y, by means of the movement means 30, so that the contact between the first abutment 101 and the second fork 260 causes a rotation of the second fork 260 around the second axis B in the first way V1, until causing contact of the third contact element 2602 and fourth contact element 2603 with the third electromagnet 231 and the fourth electromagnet 232 respectively;
b5-f. activating the third electromagnet 231 and the fourth electromagnet 232, so that the third contact element 2602 and the fourth contact element 2603 remain respectively locked in position adhering to the electromagnets 231, 232 due to the magnetic attraction, locking the second fork 250 in a first inclined position;
b5-g. moving the detection unit 20, by means of the movement means 30, to bring the first probe 252 and the second probe 262 into contact with the first lower surface R2 of the first rail R, preferably so that the central region of the weld is in an intermediate position between the first probe 252 and the second probe 262.

The step "b7" of the method can comprise one or more of the following sub-sub-steps:
b7-a. moving the detection unit 20, by means of the movement means 30, to bring the first arm 2500 and/or the second arm 2501 of the first fork 250 into contact with the second abutment 102 so that the first fork 250 and the second abutment 102 are alongside each other parallel to the second direction Y;
b7-b. moving the detection unit 20 in the second direction Y, by means of the movement means 30, so that the contact between the second abutment 102 and the first fork 250 causes a rotation of the first fork 250 around the second axis B in the second way V2, until causing contact of the first contact element 2502 and second contact element 2503 with the first electromagnet 221 and the second electromagnet 222 respectively;
b7-c. activating the first electromagnet 221 and the second electromagnet 222, so that the first contact element 2502 and the second contact element 2503 remain respectively locked in position adhering to the electromagnets 221, 222 due to the magnetic attraction, locking the first fork 250 in a second inclined position;
b7-d. moving the detection unit 20, by means of the movement means 30, to bring the third arm 2600 and/or the fourth arm 2601 of the second fork 260 into contact with the second abutment 102 so that the second fork 260 and the second abutment 102 are alongside each other parallel to the second direction Y;
b7-e. moving the detection unit 20 in the second direction Y, by means of the movement means 30, so that the contact between the second abutment 101 and the second fork 260 causes a rotation of the second fork 260 around the second axis B in the second way V2, until causing the contact of the third contact element 2602 and the fourth contact element 2603 with the third electromagnet 231 and the fourth electromagnet 232 respectively;
b7-f. activating the third electromagnet 231 and the fourth electromagnet 232, so that the third contact element 2602 and the fourth contact element 2603 remain respectively locked in position adhering to the electromagnet 231, 232 due to the magnetic attraction, locking the second fork 250 in a second inclined position;
b7-g. moving the detection unit 20, by means of the movement means 30, to bring the first probe 252 and the second probe 262 into contact with the second lower surface R3 of the first rail R, preferably so that the central region of the weld is in an intermediate position between the first probe 252 and the second probe 262.

From the above description, it is easy to see that the device 1 and the relative method of use are perfectly able to solve the drawbacks of the state of the art illustrated above.

In fact, the device 1 allows a reliable control to be performed on the entire section of the first rail R. The first part of the control, performed by means of the first probe 252 and the second probe 262, and the second part of the control, performed by means of the third probe 253, allow the identification of any defects in the head, web and central part of the foot of the first rail R. The third part of the control, performed by means of the fourth probe 263, allows more effective identification of any defects in the upper portion of the head of the first rail R. The fourth part of the control, performed by means of the first probe 252 and the second probe 262, allows the identification of any defects in a first lateral portion of the foot of the first rail R. The fifth part of the control, performed by means of the first probe 252 and the second probe 262, allows the identification of any defects in a second lateral portion of the foot of the first rail R.

It is also evident that it is possible to perform even only one of the parts of the control, or only some of them in the preferred order, if a control is required only on some portions of the section of the first rail R.

The process of control of the first rail R is therefore fully automated, and is therefore particularly reliable and reproducible.

Lastly, it is clear that modifications and variations can be made to the device and the method described here without departing from the protective scope of the present invention, which is defined solely by the appended claims.

## Claims

1. A device (1) for performing non-destructive control of a first rail (R) that develops along a first axis (A), said device (1) comprising a frame (10) designed to be moved along said first rail (R) resting at least partially on it, an ultrasound detection unit (20) supported by said frame (10), and movement means (30) designed to move said detection unit (20) with respect to said frame (10) in a first direction (X) substantially parallel, in use, to said first axis (A), in a second direction (Y) substantially perpendicular to said first direction (X) and in a third direction (Z) substantially perpendicular to said first direction (X) and said second direction (Y), **characterized in that** said detection unit (20) comprises a support (21), a first ultrasound probe (252) connected to said support (21) and a second ultrasound probe (262) connected to said support (21), said first probe (252) and said second probe (262) being of the phased array type and having respectively a first emission direction (M) and a second emission direction (N) intersecting with each other, said detection unit (20) comprising a third ultrasound probe (253) of the conventional type with a single element and/or a fourth ultrasound probe (263) of conventional type with double element, said third probe (253) and said fourth probe (263) having respectively a third emission direction (P) and a fourth emission direction (Q) substantially perpendicular to said first direction (X).

2. The device (1) according to the preceding claim, **characterized in that** said movement means (30) comprise a cross member (31) supported by said frame (10) and movable with respect to said frame (10) in said second direction (Y), a slide (32) supported by said cross member (31) and movable with respect to said cross member (31) in said first direction (X), a carriage (33) supported by said slide (32) and movable with respect to said slide (32) in said third direction (Z), and actuating means (34) designed to move said cross member (31), said slide (32) and said carriage (33), said detection unit (20) being supported by said carriage (33).

3. The device (1) according to any one of the preceding claims, **characterized in that** said first probe (252) and/or said second probe (262) are movable along said support (21).

4. The device (1) according to any one of the preceding claims, **characterized in that** said first probe (252) and/or said second probe (262) are rotatable around a second axis (B) substantially parallel to said first direction (X).

5. The device (1) according to any one of the preceding claims, **characterized in that** it comprises identification means (40) supported by said frame (10) and designed to identify a given position along said first rail (R) in which to perform said control.

6. The device (1) according to any one of the preceding claims, **characterized in that** it comprises electronic control means supported by said frame (10) and designed to control the operation of said detection unit (20) and the activation of said movement means (30).

7. The device (1) according to the preceding claim, **characterized in that** said electronic control means are designed to receive data from said detection unit (20) and to send said data to a remote operations centre.

8. A method for performing non-destructive control of a first rail (R) which develops along a first axis (A), by means of a device (1) according to any one of the claims from 1 to 7, comprising the following steps:
a. moving said device (1) along said first rail (R) until it reaches a position definable according to the need;
b1. moving said detection unit (20) in said first direction (X), second direction (Y) and third direction (Z) by means of said movement means (30) to bring said first probe (252) and second probe (262) into contact with an upper surface (R1) of said first rail (R);
b2. moving said detection unit (20) in said first direction (X) and/or in said second direction (Y) by means of said movement means (30), to move said first probe (252) and second probe (262) along said upper surface (R1) and to perform at least a first part of said control by means of said first probe (252) and second probe (262);
b3. moving said detection unit (20) in said first direction (X), second direction (Y) and third direction (Z) by means of said movement means (30) to bring said third probe (253) and/or said fourth probe (263) into contact with an upper surface (R1) of said first rail (R);
b4. moving said detection unit (20) in said first direction (X) and/or in said second direction (Y) by means of said movement means (30), to move said third probe (253) and/or said fourth probe (263) along said upper surface (R1) and to perform a second part and/or a third part of said control by means of said third probe (253) and/or said fourth probe (263).

9. A method for performing the non-destructive control of a first rail (R) which develops along a first axis (A), by means of a device (1) according to claim 4, comprising the following steps:
a. moving said device (1) along said first rail (R) until it reaches a position definable according to the need;
b1. moving said detection unit (20) in said first direction (X), second direction (Y) and third direction (Z) by means of said movement means (30) to bring said first probe (252) and second probe (262) into contact with an upper surface (R1) of said first rail (R);
b2. moving said detection unit (20) in said first direction (X) and/or in said second direction (Y) by means of said movement means (30), to move said first probe (252) and second probe (262) along said upper surface (R1) and to perform at least a first part of said control by means of said first probe (252) and second probe (262);
b3. moving said detection unit (20) in said first direction (X), second direction (Y) and third direction (Z) by means of said movement means (30) to bring said third probe (253) and/or said fourth probe (263) into contact with an upper surface (R1) of said first rail (R);
b4. moving said detection unit (20) in said first direction (X) and/or in said second direction (Y) by means of said movement means (30), to move said third probe (253) and/or said fourth probe (263) along said upper surface (R1) and to perform a second part and/or a third part of said control by means of said third probe (253) and/or said fourth probe (263);
b5. moving said detection unit (20) in said first direction (X), second direction (Y) and third direction (Z) by means of said movement means (30) and rotating said first probe (252) and second probe (262) around said second axis (B) in the first direction (V1), to bring said first probe (252) and second probe (262) into contact with a first lower surface (R2) of said first rail (R);
b6. moving said detection unit (20) in said first direction (X) and/or in said second direction (Y) by means of said movement means (30), to move said first probe (252) and second probe (262) along said first lower surface (R2) and to perform a fourth part of said control by means of said first probe (252) and second probe (262).

10. The method according to the preceding claim, **characterized in that** it further comprises the following steps:
b7. moving said detection unit (20) by means of said movement means (30) and rotating said first probe (252) and second probe (262) around said second axis (B) in the second direction (V2), to bring said first probe (252) and second probe (262) into contact with a second lower surface (R3) of said first rail (R);
b8. moving said detection unit (20) in said first direction (X) and/or in said second direction (Y) by means of said movement means (30), to move said first probe (252) and second probe (262) along said second lower surface (R3) and to perform a fifth part of said control by means of said first probe (252) and second probe (262).

11. A method for performing non-destructive control of a first rail (R) that develops along a first axis (A), by means of a device (1) according to claim 5, comprising the following steps:
a1. moving said device (1) along said first rail (R) until it reaches a welding between two profiles that compose said first rail (R);
a2. identifying a central region of said welding by means of said identification means (40);
b1. moving said detection unit (20) in said first direction (X), second direction (Y) and third direction (Z) by means of said movement means (30) to bring said first probe (252) and second probe (262) into contact with an upper surface (R1) of said first rail (R) so that said central region is in an intermediate position between said first probe (252) and said second probe (262);
b2. moving said detection unit (20) in said first direction (X) and/or in said second direction (Y) by means of said movement means (30), to move said first probe (252) and second probe (262) along said upper surface (R1) and to perform at least a first part of said control by means of said first probe (252) and second probe (262);
b3. moving said detection unit (20) in said first direction (X), second direction (Y) and third direction (Z) by means of said movement means (30) to bring said third probe (253) and/or said fourth probe (263) into contact with an upper surface (R1) of said first rail (R);
b4. moving said detection unit (20) in said first direction (X) and/or in said second direction (Y) by means of said movement means (30), to move said third probe (253) and/or said fourth probe (263) along said upper surface (R1) and to perform a second part and/or a third part of said control by means of said third probe (253) and/or said fourth probe (263).

## Patentansprüche

1. Eine Vorrichtung (1) zum Durchführen einer zerstörungsfreien Prüfung einer ersten Schiene (R), die sich entlang einer ersten Achse (A) erstreckt, wobei die Vorrichtung (1) Folgendes beinhaltet: einen Rahmen (10), der ausgelegt ist, um entlang der ersten Schiene (R) bewegt zu werden, und der zumindest teilweise auf dieser ruht, eine Ultraschalldetektionseinheit (20), die von dem Rahmen (10) getragen wird, und ein Bewegungsmittel (30), das ausgelegt ist, um die Detektionseinheit (20) in Bezug auf den Rahmen (10) in eine erste Richtung (X), die im Gebrauch im Wesentlichen parallel zu der ersten Achse (A) ist, in eine zweite Richtung (Y), die im Wesentlichen senkrecht zu der ersten Richtung (X) ist, und in eine dritte Richtung (Z), die im Wesentlichen senkrecht zu der ersten Richtung (X) und der zweiten Richtung (Y) ist, zu bewegen, **dadurch gekennzeichnet, dass** die Detektionseinheit (20) einen Träger (21), eine erste Ultraschallsonde (252), die mit dem Träger (21) verbunden ist, und eine zweite Ultraschallsonde (262), die mit dem Träger (21) verbunden ist, beinhaltet, wobei die erste Sonde (252) und die zweite Sonde (262) vom Phased-Array-Typ sind und jeweils eine erste Emissionsrichtung (M) und eine zweite Emissionsrichtung (N) aufweisen, die sich schneiden, wobei die Detektionseinheit (20) eine dritte Ultraschallsonde (253) vom herkömmlichen Typ mit einem einzigen Element und/oder eine vierte Ultraschallsonde (263) vom herkömmlichen Typ mit einem doppelten Element beinhaltet, wobei die dritte Sonde (253) und die vierte Sonde (263) jeweils eine dritte Emissionsrichtung (P) und eine vierte Emissionsrichtung (Q) aufweisen, die im Wesentlichen senkrecht zu der ersten Richtung (X) sind.

2. Vorrichtung (1) gemäß dem vorhergehenden Anspruch, **dadurch gekennzeichnet, dass** das Bewegungsmittel (30) Folgendes beinhaltet: ein Querelement (31), das von dem Rahmen (10) getragen wird und in Bezug auf den Rahmen (10) in der zweiten Richtung (Y) beweglich ist, einen Schieber (32), der von dem Querelement (31) getragen wird und in Bezug auf das Querelement (31) in der ersten Richtung (X) beweglich ist, einen Schlitten (33), der von dem Schieber (32) getragen wird und in Bezug auf den Schieber (32) in der dritten Richtung (Z) beweglich ist, und ein Betätigungsmittel (34), das ausgelegt ist, um das Querelement (31), den Schieber (32) und den Schlitten (33) zu bewegen, wobei die Detektionseinheit (20) von dem Schlitten (33) getragen wird.

3. Vorrichtung (1) gemäß einem der vorhergehenden Ansprüche, **dadurch gekennzeichnet, dass** die erste Sonde (252) und/oder die zweite Sonde (262) entlang des Trägers (21) beweglich sind.

4. Vorrichtung (1) gemäß einem der vorhergehenden Ansprüche, **dadurch gekennzeichnet, dass** die erste Sonde (252) und/oder die zweite Sonde (262) um eine zweite Achse (B) drehbar sind, die im Wesentlichen parallel zu der ersten Richtung (X) ist.

5. Vorrichtung (1) gemäß einem der vorhergehenden Ansprüche, **dadurch gekennzeichnet, dass** sie ein Identifikationsmittel (40) beinhaltet, das von dem Rahmen (10) getragen wird und ausgelegt ist, um eine gegebene Position entlang der ersten Schiene (R) zu identifizieren, in der die Prüfung durchgeführt werden soll.

6. Vorrichtung (1) gemäß einem der vorhergehenden Ansprüche, **dadurch gekennzeichnet, dass** sie ein elektronisches Steuermittel beinhaltet, das von dem Rahmen (10) getragen wird und ausgelegt ist, um den Betrieb der Detektionseinheit (20) und die Aktivierung des Bewegungsmittels (30) zu steuern.

7. Vorrichtung (1) gemäß dem vorhergehenden Anspruch, **dadurch gekennzeichnet, dass** das elektronische Steuermittel ausgelegt ist, um Daten von der Detektionseinheit (20) zu empfangen und um die Daten an ein entferntes Betriebszentrum zu senden.

8. Ein Verfahren zum Durchführen einer zerstörungsfreien Prüfung einer ersten Schiene (R), die sich entlang einer ersten Achse (A) erstreckt, mittels einer Vorrichtung (1) gemäß einem der Ansprüche 1 bis 7, das die folgenden Schritte beinhaltet:
a. Bewegen der Vorrichtung (1) entlang der ersten Schiene (R), bis sie eine je nach Bedarf definierbare Position erreicht;
b1. Bewegen der Detektionseinheit (20) in die erste Richtung (X), die zweite Richtung (Y) und die dritte Richtung (Z) mittels des Bewegungsmittels (30), um die erste Sonde (252) und die zweite Sonde (262) in Kontakt mit einer oberen Oberfläche (R1) der ersten Schiene (R) zu bringen;
b2. Bewegen der Detektionseinheit (20) in die erste Richtung (X) und/oder in die zweite Richtung (Y) mittels des Bewegungsmittels (30), um die erste Sonde (252) und die zweite Sonde (262) entlang der oberen Oberfläche (R1) zu bewegen und um zumindest einen ersten Teil der Prüfung mittels der ersten Sonde (252) und der zweiten Sonde (262) durchzuführen;
b3. Bewegen der Detektionseinheit (20) in die erste Richtung (X), die zweite Richtung (Y) und die dritte Richtung (Z) mittels des Bewegungsmittels (30), um die dritte Sonde (253) und/oder die vierte Sonde (263) in Kontakt mit einer oberen Oberfläche (R1) der ersten Schiene (R) zu bringen;
b4. Bewegen der Detektionseinheit (20) in die erste Richtung (X) und/oder in die zweite Richtung (Y) mittels des Bewegungsmittels (30), um die dritte Sonde (253) und/oder die vierte Sonde (263) entlang der oberen Oberfläche (R1) zu bewegen und um einen zweiten Teil und/oder einen dritten Teil der Prüfung mittels der dritten Sonde (253) und/oder der vierten Sonde (263) durchzuführen.

9. Ein Verfahren zum Durchführen der zerstörungsfreien Prüfung einer ersten Schiene (R), die sich entlang einer ersten Achse (A) erstreckt, mittels einer Vorrichtung (1) gemäß Anspruch 4, das die folgenden Schritte beinhaltet:
a. Bewegen der Vorrichtung (1) entlang der ersten Schiene (R), bis sie eine je nach Bedarf definierbare Position erreicht;
b1. Bewegen der Detektionseinheit (20) in die erste Richtung (X), die zweite Richtung (Y) und die dritte Richtung (Z) mittels des Bewegungsmittels (30), um die erste Sonde (252) und die zweite Sonde (262) in Kontakt mit einer oberen Oberfläche (R1) der ersten Schiene (R) zu bringen;
b2. Bewegen der Detektionseinheit (20) in die erste Richtung (X) und/oder in die zweite Richtung (Y) mittels des Bewegungsmittels (30), um die erste Sonde (252) und die zweite Sonde (262) entlang der oberen Oberfläche (R1) zu bewegen und um zumindest einen ersten Teil der Prüfung mittels der ersten Sonde (252) und der zweiten Sonde (262) durchzuführen;
b3. Bewegen der Detektionseinheit (20) in die erste Richtung (X), die zweite Richtung (Y) und die dritte Richtung (Z) mittels des Bewegungsmittels (30), um die dritte Sonde (253) und/oder die vierte Sonde (263) in Kontakt mit einer oberen Oberfläche (R1) der ersten Schiene (R) zu bringen;
b4. Bewegen der Detektionseinheit (20) in die erste Richtung (X) und/oder in die zweite Richtung (Y) mittels des Bewegungsmittels (30), um die dritte Sonde (253) und/oder die vierte Sonde (263) entlang der oberen Oberfläche (R1) zu bewegen und um einen zweiten Teil und/oder einen dritten Teil der Prüfung mittels der dritten Sonde (253) und/oder der vierten Sonde (263) durchzuführen;
b5. Bewegen der Detektionseinheit (20) in die erste Richtung (X), die zweite Richtung (Y) und die dritte Richtung (Z) mittels des Bewegungsmittels (30) und Drehen der ersten Sonde (252) und der zweiten Sonde (262) um die zweite Achse (B) in die erste Richtung (V1), um die erste Sonde (252) und die zweite Sonde (262) in Kontakt mit einer ersten unteren Oberfläche (R2) der ersten Schiene (R) zu bringen;
b6. Bewegen der Detektionseinheit (20) in die erste Richtung (X) und/oder in die zweite Richtung (Y) mittels des Bewegungsmittels (30), um die erste Sonde (252) und die zweite Sonde (262) entlang der ersten unteren Oberfläche (R2) zu bewegen und um einen vierten Teil der Prüfung mittels der ersten Sonde (252) und der zweiten Sonde (262) durchzuführen.

10. Verfahren gemäß dem vorhergehenden Anspruch, **dadurch gekennzeichnet, dass** es ferner die folgenden Schritte beinhaltet:
b7. Bewegen der Detektionseinheit (20) mittels des Bewegungsmittels (30) und Drehen der ersten Sonde (252) und der zweiten Sonde (262) um die zweite Achse (B) in die zweite Richtung (V2), um die erste Sonde (252) und die zweite Sonde (262) in Kontakt mit einer zweiten unteren Oberfläche (R3) der ersten Schiene (R) zu bringen;
b8. Bewegen der Detektionseinheit (20) in die erste Richtung (X) und/oder in die zweite Richtung (Y) mittels des Bewegungsmittels (30), um die erste Sonde (252) und die zweite Sonde (262) entlang der zweiten unteren Oberfläche (R3) zu bewegen und um einen fünften Teil der Prüfung mittels der ersten Sonde (252) und der zweiten Sonde (262) durchzuführen.

11. Ein Verfahren zum Durchführen einer zerstörungsfreien Prüfung einer ersten Schiene (R), die sich entlang einer ersten Achse (A) erstreckt, mittels einer Vorrichtung (1) gemäß Anspruch 5, das die folgenden Schritte beinhaltet:
a1. Bewegen der Vorrichtung (1) entlang der ersten Schiene (R), bis sie eine Schweißung zwischen zwei Profilen, die die erste Schiene (R) bilden, erreicht;
a2. Identifizieren eines zentralen Bereichs der Schweißung mittels des Identifikationsmittels (40);
b1. Bewegen der Detektionseinheit (20) in die erste Richtung (X), die zweite Richtung (Y) und die dritte Richtung (Z) mittels des Bewegungsmittels (30), um die erste Sonde (252) und die zweite Sonde (262) in Kontakt mit einer oberen Oberfläche (R1) der ersten Schiene (R) zu bringen, sodass sich der zentrale Bereich in einer Zwischenposition zwischen der ersten Sonde (252) und der zweiten Sonde (262) befindet;
b2. Bewegen der Detektionseinheit (20) in die erste Richtung (X) und/oder in die zweite Richtung (Y) mittels des Bewegungsmittels (30), um die erste Sonde (252) und die zweite Sonde (262) entlang der oberen Oberfläche (R1) zu bewegen und um zumindest einen ersten Teil der Prüfung mittels der ersten Sonde (252) und der zweiten Sonde (262) durchzuführen;
b3. Bewegen der Detektionseinheit (20) in die erste Richtung (X), die zweite Richtung (Y) und die dritte Richtung (Z) mittels des Bewegungsmittels (30), um die dritte Sonde (253) und/oder die vierte Sonde (263) in Kontakt mit einer oberen Oberfläche (R1) der ersten Schiene (R) zu bringen;
b4. Bewegen der Detektionseinheit (20) in die erste Richtung (X) und/oder in die zweite Richtung (Y) mittels des Bewegungsmittels (30), um die dritte Sonde (253) und/oder die vierte Sonde (263) entlang der oberen Oberfläche (R1) zu bewegen und um einen zweiten Teil und/oder einen dritten Teil der Prüfung mittels der dritten Sonde (253) und/oder der vierten Sonde (263) durchzuführen.

## Revendications

1. Un dispositif (1) pour effectuer un contrôle non destructif d'un premier rail (R) qui se développe le long d'un premier axe (A), ledit dispositif (1) comprenant un châssis (10) conçu pour être déplacé le long dudit premier rail (R) reposant au moins partiellement sur celui-ci, une unité de détection ultrasonore (20) supportée par ledit châssis (10), et des moyens de déplacement (30) conçus pour déplacer ladite unité de détection (20) par rapport audit châssis (10) dans une première direction (X) substantiellement parallèle, lors de l'utilisation, audit premier axe (A), dans une deuxième direction (Y) substantiellement perpendiculaire à ladite première direction (X) et dans une troisième direction (Z) substantiellement perpendiculaire à ladite première direction (X) et à ladite deuxième direction (Y),
**caractérisé en ce que** ladite unité de détection (20) comprend un support (21), une première sonde ultrasonore (252) reliée audit support (21) et une deuxième sonde ultrasonore (262) reliée audit support (21), ladite première sonde (252) et ladite deuxième sonde (262) étant du type réseau à commande de phase et ayant respectivement une première direction d'émission (M) et une deuxième direction d'émission (N) se coupant l'une l'autre, ladite unité de détection (20) comprenant une troisième sonde ultrasonore (253) du type conventionnel avec un seul élément et/ou une quatrième sonde ultrasonore (263) de type conventionnel avec un double élément, ladite troisième sonde (253) et ladite quatrième sonde (263) ayant respectivement une troisième direction d'émission (P) et une quatrième direction d'émission (Q) substantiellement perpendiculaires à ladite première direction (X).

2. Le dispositif (1) selon la revendication précédente, **caractérisé en ce que** lesdits moyens de déplacement (30) comprennent une traverse (31) supportée par ledit châssis (10) et mobile par rapport audit châssis (10) dans ladite deuxième direction (Y), une coulisse (32) supportée par ladite traverse (31) et mobile par rapport à ladite traverse (31) dans ladite première direction (X), un chariot (33) supporté par ladite coulisse (32) et mobile par rapport à ladite coulisse (32) dans ladite troisième direction (Z), et des moyens d'actionnement (34) conçus pour déplacer ladite traverse (31), ladite coulisse (32) et ledit chariot (33), ladite unité de détection (20) étant supportée par ledit chariot (33).

3. Le dispositif (1) selon l'une quelconque des revendications précédentes, **caractérisé en ce que** ladite première sonde (252) et/ou ladite deuxième sonde (262) sont mobiles le long dudit support (21).

4. Le dispositif (1) selon l'une quelconque des revendications précédentes, **caractérisé en ce que** ladite première sonde (252) et/ou ladite deuxième sonde (262) sont aptes à tourner autour d'un deuxième axe (B) substantiellement parallèle à ladite première direction (X).

5. Le dispositif (1) selon l'une quelconque des revendications précédentes, **caractérisé en ce qu'**il comprend des moyens d'identification (40) supportés par ledit châssis (10) et conçus pour identifier une position donnée le long dudit premier rail (R) dans laquelle **il** convient d'effectuer ledit contrôle.

6. Le dispositif (1) selon l'une quelconque des revendications précédentes, **caractérisé en ce qu'**il comprend des moyens de contrôle électroniques supportés par ledit châssis (10) et conçus pour contrôler le fonctionnement de ladite unité de détection (20) et l'activation desdits moyens de déplacement (30).

7. Le dispositif (1) selon la revendication précédente, **caractérisé en ce que** lesdits moyens de contrôle électroniques sont conçus pour recevoir des données en provenance de ladite unité de détection (20) et pour envoyer lesdites données à un centre d'opérations à distance.

8. Un procédé pour effectuer un contrôle non destructif d'un premier rail (R) qui se développe le long d'un premier axe (A), au moyen d'un dispositif (1) selon l'une quelconque des revendications allant de 1 à 7, comprenant les étapes suivantes :
a. le déplacement dudit dispositif (1) le long dudit premier rail (R) jusqu'à ce qu'il atteigne une position définissable selon le besoin ;
b1. le déplacement de ladite unité de détection (20) dans lesdites première direction (X), deuxième direction (Y) et troisième direction (Z) au moyen desdits moyens de déplacement (30) pour amener lesdites première sonde (252) et deuxième sonde (262) en contact avec une surface supérieure (R1) dudit premier rail (R) ;
b2. le déplacement de ladite unité de détection (20) dans ladite première direction (X) et/ou dans ladite deuxième direction (Y) au moyen desdits moyens de déplacement (30), pour déplacer lesdites première sonde (252) et deuxième sonde (262) le long de ladite surface supérieure (R1) et pour effectuer au moins une première partie dudit contrôle au moyen desdites première sonde (252) et deuxième sonde (262) ;
b3. le déplacement de ladite unité de détection (20) dans lesdites première direction (X), deuxième direction (Y) et troisième direction (Z) au moyen desdits moyens de déplacement (30) pour amener ladite troisième sonde (253) et/ou ladite quatrième sonde (263) en contact avec une surface supérieure (R1) dudit premier rail (R) ;
b4. le déplacement de ladite unité de détection (20) dans ladite première direction (X) et/ou dans ladite deuxième direction (Y) au moyen desdits moyens de déplacement (30), pour déplacer ladite troisième sonde (253) et/ou ladite quatrième sonde (263) le long de ladite surface supérieure (R1) et pour effectuer une deuxième partie et/ou une troisième partie dudit contrôle au moyen de ladite troisième sonde (253) et/ou de ladite quatrième sonde (263).

9. Un procédé pour effectuer le contrôle non destructif d'un premier rail (R) qui se développe le long d'un premier axe (A), au moyen d'un dispositif (1) selon la revendication 4, comprenant les étapes suivantes :
a. le déplacement dudit dispositif (1) le long dudit premier rail (R) jusqu'à ce qu'il atteigne une position définissable selon le besoin ;
b1. le déplacement de ladite unité de détection (20) dans lesdites première direction (X), deuxième direction (Y) et troisième direction (Z) au moyen desdits moyens de déplacement (30) pour amener lesdites première sonde (252) et deuxième sonde (262) en contact avec une surface supérieure (R1) dudit premier rail (R) ;
b2. le déplacement de ladite unité de détection (20) dans ladite première direction (X) et/ou dans ladite deuxième direction (Y) au moyen desdits moyens de déplacement (30), pour déplacer lesdites première sonde (252) et deuxième sonde (262) le long de ladite surface supérieure (R1) et pour effectuer au moins une première partie dudit contrôle au moyen desdites première sonde (252) et deuxième sonde (262) ;
b3. le déplacement de ladite unité de détection (20) dans lesdites première direction (X), deuxième direction (Y) et troisième direction (Z) au moyen desdits moyens de déplacement (30) pour amener ladite troisième sonde (253) et/ou ladite quatrième sonde (263) en contact avec une surface supérieure (R1) dudit premier rail (R) ;
b4. le déplacement de ladite unité de détection (20) dans ladite première direction (X) et/ou dans ladite deuxième direction (Y) au moyen desdits moyens de déplacement (30), pour déplacer ladite troisième sonde (253) et/ou ladite quatrième sonde (263) le long de ladite surface supérieure (R1) et pour effectuer une deuxième partie et/ou une troisième partie dudit contrôle au moyen de ladite troisième sonde (253) et/ou de ladite quatrième sonde (263) ;
b5. le déplacement de ladite unité de détection (20) dans lesdites première direction (X), deuxième direction (Y) et troisième direction (Z) au moyen desdits moyens de déplacement (30) et la rotation desdites première sonde (252) et deuxième sonde (262) autour dudit deuxième axe (B) dans la première direction (V1), pour amener lesdites première sonde (252) et deuxième sonde (262) en contact avec une première surface inférieure (R2) dudit premier rail (R) ;
b6. le déplacement de ladite unité de détection (20) dans ladite première direction (X) et/ou dans ladite deuxième direction (Y) au moyen desdits moyens de déplacement (30), pour déplacer lesdites première sonde (252) et deuxième sonde (262) le long de ladite première surface inférieure (R2) et pour effectuer une quatrième partie dudit contrôle au moyen desdites première sonde (252) et deuxième sonde (262).

10. Le procédé selon la revendication précédente, **caractérisé en ce qu'**il comprend en outre les étapes suivantes :
b7. le déplacement de ladite unité de détection (20) au moyen desdits moyens de déplacement (30) et la rotation desdites première sonde (252) et deuxième sonde (262) autour dudit deuxième axe (B) dans la deuxième direction (V2), pour amener lesdites première sonde (252) et deuxième sonde (262) en contact avec une deuxième surface inférieure (R3) dudit premier rail (R) ;
b8. le déplacement de ladite unité de détection (20) dans ladite première direction (X) et/ou dans ladite deuxième direction (Y) au moyen desdits moyens de déplacement (30), pour déplacer lesdites première sonde (252) et deuxième sonde (262) le long de ladite deuxième surface inférieure (R3) et pour effectuer une cinquième partie dudit contrôle au moyen desdites première sonde (252) et deuxième sonde (262).

11. Un procédé pour effectuer un contrôle non destructif d'un premier rail (R) qui se développe le long d'un premier axe (A), au moyen d'un dispositif (1) selon la revendication 5, comprenant les étapes suivantes :
a1. le déplacement dudit dispositif (1) le long dudit premier rail (R) jusqu'à ce qu'il atteigne une soudure entre deux profils qui composent ledit premier rail (R) ;
a2. l'identification d'une région centrale de ladite soudure au moyen desdits moyens d'identification (40) ;
b1. le déplacement de ladite unité de détection (20) dans lesdites première direction (X), deuxième direction (Y) et troisième direction (Z) au moyen desdits moyens de déplacement (30) pour amener lesdites première sonde (252) et deuxième sonde (262) en contact avec une surface supérieure (R1) dudit premier rail (R) de sorte que ladite région centrale se trouve dans une position intermédiaire entre ladite première sonde (252) et ladite deuxième sonde (262) ;
b2. le déplacement de ladite unité de détection (20) dans ladite première direction (X) et/ou dans ladite deuxième direction (Y) au moyen desdits moyens de déplacement (30), pour déplacer lesdites première sonde (252) et deuxième sonde (262) le long de ladite surface supérieure (R1) et pour effectuer au moins une première partie dudit contrôle au moyen desdites première sonde (252) et deuxième sonde (262) ;
b3. le déplacement de ladite unité de détection (20) dans lesdites première direction (X), deuxième direction (Y) et troisième direction (Z) au moyen desdits moyens de déplacement (30) pour amener ladite troisième sonde (253) et/ou ladite quatrième sonde (263) en contact avec une surface supérieure (R1) dudit premier rail (R) ;
b4. le déplacement de ladite unité de détection (20) dans ladite première direction (X) et/ou dans ladite deuxième direction (Y) au moyen desdits moyens de déplacement (30), pour déplacer ladite troisième sonde (253) et/ou ladite quatrième sonde (263) le long de ladite surface supérieure (R1) et pour effectuer une deuxième partie et/ou une troisième partie dudit contrôle au moyen de ladite troisième sonde (253) et/ou de ladite quatrième sonde (263).
